# FASCICULE DE BREVET EUROPEEN

(11) **EP 2 005 143 B1**
(45) Date de publication et mention de la délivrance du brevet: **14.10.2015**
(21) Numéro de dépôt: 07731179.3
(22) Date de dépôt: 23.03.2007
(51) Int. Cl.: G01N 21/64, A61B 5/00, A61B 1/04, A61B 1/07

(54) **MICROSCOPIE DE FLUORESCENCE FIBREE A BASE DE BLEU DE METHYLENE**
FASERFLUORESZENZMIKROSKOP AUF DER GRUNDLAGE VON METHYLENBLAU
METHYLENE BLUE BASED FIBRED FLUORESCENCE MICROSCOPY

(30) Priorité: 31.03.2006 FR 0602793
(43) Date de publication de la demande: 24.12.2008
(73) Titulaire: Mauna Kea Technologies, 75010 Paris (FR)
(72) Inventeur: ABRAT, Benjamin, F-75016 Paris (FR); CAVE, Charlotte, F-75001 Paris (FR); LOISEAU, Alexandre, F-75018 Paris (FR); LOPEZ, Jérôme, F-75014 Paris (FR); OSDOIT, Anne, F-75018 Paris (FR); VIELLEROBE, Bertrand, F-94130 Nogent-sur-Marne (FR)
(74) Mandataire: Pontet Allano & Associes
(86) Numéro de dépôt international: PCT/FR2007/000490
(87) Numéro de publication internationale: WO 2007/118954

(56) Documents cités:
- WO-A-2004/008952
- US-A- 6 083 487
- US-A1- 2005 182 321
- YAROSLAVSKY A N ET AL: "Dye-Enhanced Reflectance and Fluorescence Confocal Microscopy as an Optical Pathology Tool" PROC. SPIE, vol. 6091, février 2006 (2006-02), pages 609107-1-609107-7, XP002409560
- JUAN C. STOCKERT ET AL: 'Photodynamic toxicity and its prevention by antioxidative agents in Bufo arenarum embryos' TOXICOLOGY vol. 192, no. 2-3, 01 Novembre 2003, pages 211 - 218, XP055035538 DOI: 10.1016/S0300-483X(03)00334-2 ISSN: 0300-483X

## Description

La présente invention se rapporte à un procédé d'acquisition d'imagerie de fluorescence, in vivo, d'un tissu au moyen d'un système d'acquisition comprenant au moins une fibre optique d'excitation du tissu par faisceau lumineux.

La présente invention trouve une application particulièrement intéressante dans le domaine de l'imagerie médicale in vivo. Pour ce faire, on utilise un système fibré permettant d'obtenir, grâce à un traitement informatique, une image construite en temps réel point à point. Un tel système permet d'observer et d'analyser un tissu biologique in vivo in situ en temps réel, notamment accessible via le canal opérateur d'un endoscope ou intégré à l'endoscope.

D'une façon générale, un tel système peut comprendre une unité de traitement apte à générer une image à partir de signaux électriques en provenance de photo-détecteurs. Ces photodétecteurs reçoivent des signaux de fluorescence en provenance du tissu et véhiculés par au moins une fibre optique. Le système comprend également des moyens de balayage pour que le faisceau d'excitation balaye point par point le champ imagé dans le tissu.

En imagerie de fluorescence, un photon vient exciter une molécule. La désexcitation de cette dernière provoque l'émission d'un photon fluorescent. L'énergie du photon excitateur correspond exactement à la quantité d'énergie nécessaire pour porter la molécule à un état excité donné.

En général, la fluorescence observée peut provenir d'un composé exogène (typiquement un marqueur administré) ou d'un composé endogène qui est soit fabriquée par des cellules (marqueur de type transgénique) d'un tissu biologique, soit naturellement présent dans les cellules (autofluorescence). En particulier, lorsqu'on utilise un composé exogène destiné à être administré, il est généralement admis et conseillé d'utiliser un marqueur présentant un fort caractère fluorescent. On peut ainsi avantageusement utiliser un laser de faible puissance (énergie d'excitation), la quantité de photons fluorescents étant largement suffisant pour élaborer des images de grande qualité.

On utilise généralement les marqueurs suivants qui sont reconnus pour leurs propriétés de fluorescence : les rhodamines, fluorescéines, cyanine,...

Toutefois, le problème avec de tels marqueurs est qu'ils sont onéreux et pour la plupart toxiques.

La présente invention a pour but un procédé d'imagerie in vivo simplifié, peu onéreux et permettant une acquisition rapide.

On atteint au moins l'un des objectifs précités avec un procédé d'acquisition d'imagerie de fluorescence, in vivo, d'un tissu au moyen d'un système d'acquisition comprenant au moins une fibre optique d'excitation du tissu par balayage d'un faisceau lumineux. Selon l'invention, on utilise ledit système pour la détection de signaux de fluorescence émis par du Bleu de Méthylène présent dans le tissu.

La présente invention est notamment remarquable par le fait qu'on utilise le Bleu de Méthylène pour ses propriétés de fluorescence dans un système fibré pour de l'imagerie in vivo. Ce système peut notamment être de type confocal. Le caractère confocal est obtenu grâce à un filtrage spatial permettant de détecter un signal de retour provenant uniquement du point excité du tissu et empruntant le même chemin optique que le signal d'excitation.

L'utilisation du Bleu de Méthylène, ou chlorure de méthylthioninium, va à l'encontre d'un préjugé généralement admis selon lequel le marqueur doit posséder de grandes capacités de fluorescence. Au contraire dans le procédé selon la présente invention, on a privilégié la simplicité d'utilisation plutôt que l'efficacité du marqueur en terme de fluorescence. Ce procédé permet de simplifier le processus d'acquisition d'imagerie de fluorescence par utilisation d'une substance qui est couramment utilisée dans le domaine médical mais pour des usages différents. En effet, les propriétés pharmacologiques pour lesquels on utilise généralement le Bleu de Méthylène sont les suivantes : antidote, colorant, antiseptique, ou encore agent diagnostique.

Par ailleurs, et plus précisément, le bleu de méthylène est utilisé en endoscopie comme "colorant vital". Cela s'appelle alors de la "chromoendoscopie" ou "chromoscopie". Le bleu de méthylène est aussi utilisé comme agent de contraste dans une nouvelle technique appelée "Endocytoscopy". Dans ce cas, il est seulement utilisé comme marqueur. Le bleu de méthylène est aussi utilisé comme agent de contraste pour le photodiagnostic.

L'utilisation des propriétés de fluorescence du bleu de méthylène est décrite dans A. N. Yaroslavsky et al., "Dye-Enhanced Reflectance and Fluorescence Confocal Microscopy as an Optical Pathology Tool", Proc. of SPIE, vol. 6091, pages 609107-1 à 609107-7, 2006.

Le Bleu de Méthylène est donc couramment utilisé, notamment en tant que substance antiseptique. A ce titre, on peut donc utiliser le Bleu de Méthylène sur un tissu lors d'une étape préalable de traitement antiseptique, et profiter avantageusement de sa présence sur le tissu pour acquérir une image de fluorescence du tissu. On voit tout de suite les avantages, en terme de coût et de délai, qui découlent du procédé puisqu'il n'est plus nécessaire d'administrer un autre fluorophore.

Le procédé selon l'invention est défini dans la revendication 1.

Selon un mode de mise en oeuvre avantageux de l'invention, le système d'acquisition utilisé est un système d'endo-microscopie confocale (microscopie pendant l'endoscopie).

Suivant l'invention, le faisceau lumineux d'excitation est un faisceau laser. En outre, la longueur d'onde du faisceau lumineux d'excitation peut être comprise entre 600nm et 680nm. L'utilisation d'un système d'endomicroscopie confocale avec une longueur d'onde adéquate permet d'obtenir des images au niveau microscopique.

Avantageusement, lors d'une utilisation sur des tissus humains notamment, la concentration du Bleu de Méthylène peut être sensiblement égale à 0.5%. On préconise une telle valeur particulièrement pour lutter contre la toxicité du Bleu de Méthylène. Cependant, dans le cas présent, en exploitant au mieux les propriétés de fluorescence du Bleu de Méthylène par éclairage laser notamment, il se crée de la phototoxicité. Pour lutter contre cela, on peut aussi prévoir l'utilisation d'une concentration maximum du Bleu de Méthylène égale à 0.5%. Dans ce cas, la puissance du faisceau laser d'excitation et la sensibilité du système d'acquisition sont adaptées de façon à détecter un signal de fluorescence de niveau suffisamment élevé pour permettre au système d'acquisition d'élaborer une image. Selon l'invention, la puissance du faisceau laser d'excitation est inférieure ou égale à 10 mW pour une microseconde d'éclairage. Plus précisément, la puissance du laser est adaptée pour ne pas atteindre le seuil de phototoxicité du bleu de méthylène, typiquement entre 1 et 5 mW sur le tissu.

Hors de l'invention telle que défini dans la revendication 1, on peut avantageusement utiliser des concentrations de bleu de méthylène entre 0.1% et 0.01% pour une puissance laser comprise entre 10 et 15 mW. Ceci permet notamment encore d'obtenir des images de bonne qualité.

Selon une caractéristique avantageuse de l'invention, le système d'acquisition est équipé d'un guide d'image constitué d'une pluralité de fibres optiques éclairées tour à tour, chaque fibre étant apte à véhiculer le signal d'excitation et le signal de fluorescence rétro-émis. Le système selon l'invention permet la réalisation d'une image de fluorescence in vivo, in situ, déportée avec une résolution microscopique. Le guide d'image ou "fiber bundle" en langue anglaise, présente une flexibilité et une taille permettant une application en endoscopie notamment par insertion dans un canal opérateur. Le champ à imager dans le tissu peut être balayé point à point ou ligne par ligne par le signal d'excitation.

De préférence, le faisceau lumineux balaye le tissu de façon à acquérir en temps réel au moins douze images par seconde.

Suivant un autre aspect de l'invention, il est proposé une application du procédé tel que défini ci-dessus pour l'acquisition d'images de noyaux cellulaires en endomicroscopie confocale.

La présente invention sera mieux comprise et d'autres avantages apparaîtront à la lumière de la description qui va suivre d'un exemple de réalisation, description faite en référence aux dessins annexés sur lesquels :
- la figure 1 représente schématiquement ledit exemple choisi de réalisation ; et
- la figure 2 est un schéma fonctionnel de l'appareillage selon l'invention.

Bien que l'invention n'y soit pas limitée, on va maintenant décrire un système d'imagerie microscopique confocale de fluorescence à base du bleu de méthylène. Le système d'acquisition peut consister en l'un des appareils (avec ou sans tête optique) décrits dans la demande de brevet WO 2004/008952. Mais on peut aussi utiliser tout autre appareil de fluorescence adapté.

L'élément 13 est un tissu biologique dans lequel on a injecté du bleu de méthylène de façon à marquer des noyaux cellulaires. Pour un tissu humain par exemple, on prend soin de ne pas dépasser la concentration de 0.5%.

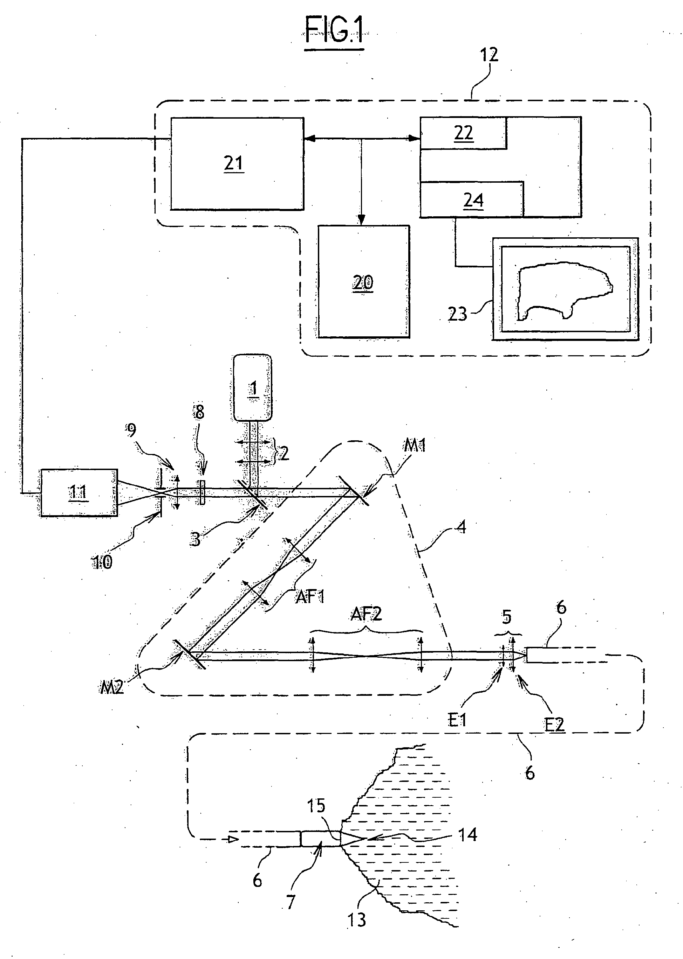

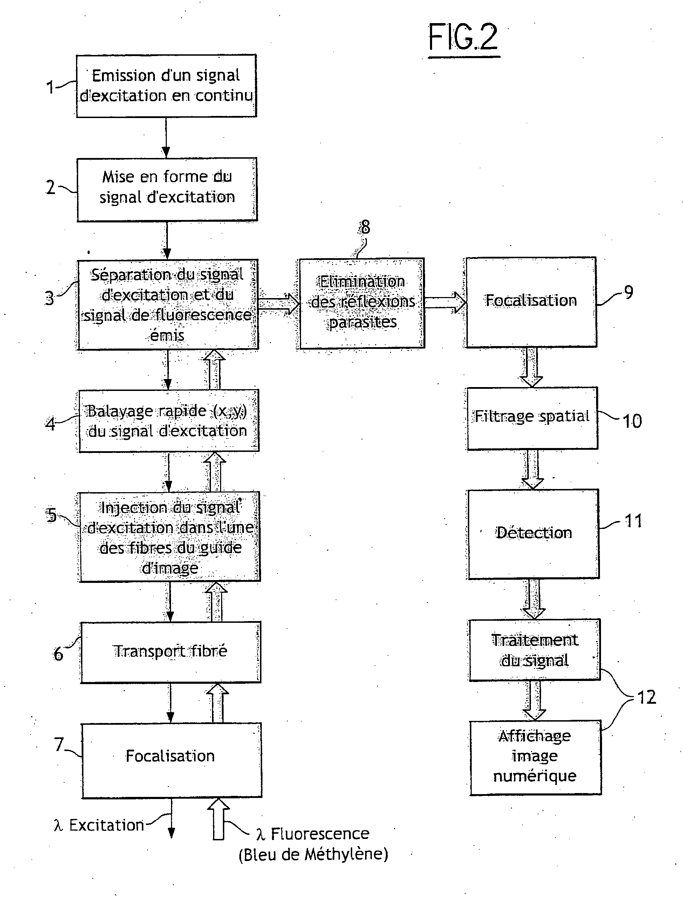

Selon l'exemple choisi et représenté sur les figures 1 et 2, l'appareillage comporte :
- une source lumineuse 1 ;
- des moyens 2 de mise en forme du faisceau d'excitation ;
- des moyens 3 de séparation de longueurs d'onde ;
- des moyens 4 de balayage ;
- des moyens 5 d'injection de faisceau ;
- un guide d'image 6 constitué de fibres optiques souples ;
- une tête optique de focalisation 7 ;
- des moyens 8 de réjection du faisceau d'excitation ;
- des moyens 9 de focalisation du signal de fluorescence ;
- des moyens 10 de filtrage spatial du signal de fluorescence ;
- des moyens 11 de détection du signal de fluorescence ; et
- des moyens 12 de traitement électronique et informatique du signal de fluorescence détecté et de visualisation.

Ces différents éléments sont détaillés ci-après.

La source lumineuse 1 est un laser émettant à une longueur d'onde d'excitation comprise entre 600nm et 680nm permettant d'exciter le bleu de méthylène, par exemple 635nm. Pour optimiser l'injection dans l'une des fibres du guide d'image 6, le faisceau d'excitation est circulaire pour pouvoir injecter une fibre de section également circulaire et, pour optimiser le taux d'injection, le laser est de préférence monomode longitudinal pour présenter le meilleur front d'onde possible pour l'injection dans une fibre optique faiblement multimode. Le laser émet de manière continue et stable (bruit le plus faible possible, <1%). La puissance en sortie disponible est de l'ordre de 20mW. A titre d'exemples, on peut utiliser un laser à puits quantiques (VCSEL), une diode laser ou encore un laser à gaz tel que l'hélium-néon (He-Ne).

En sortie de la source 1, sont placés les moyens 2 de mise en forme du faisceau laser d'excitation. Ils sont constitués d'un système optique afocal de grandissement différent de 1, composé de deux lentilles qui permettent de modifier le diamètre du faisceau laser. Le grandissement est calculé de sorte que le diamètre du faisceau soit adapté aux moyens d'injection 5 dans une fibre.

Le faisceau laser d'excitation remis en forme est ensuite dirigé vers les moyens 3 prévus pour séparer les longueurs d'ondes d'excitation et de fluorescence. Il s'agit par exemple d'un filtre dichroïque ayant une efficacité de transmission de 98 à 99 % de la longueur d'onde d'excitation et qui réfléchit donc sensiblement les autres longueurs d'onde. Le signal de fluorescence, empruntant au retour le même chemin optique que le signal d'excitation (caractère confocal), sera ainsi envoyé pratiquement totalement vers la voie de détection (8-11). Les moyens de réjection 8 placés sur la voie de détection servent à éliminer totalement les 1 à 2 % de réflexions parasites à la longueur d'onde d'excitation 635 nm qui passent vers la voie de détection (par exemple un filtre de réjection à 635 nm ou un filtre passe bande ne permettant par exemple qu'une transmission entre 640 et 800 nm).

Les moyens de balayage 4 reprennent ensuite le faisceau d'excitation. Selon l'exemple choisi et représenté sur la figure 1, ces moyens comprennent un miroir M1 résonant à 4 KHz servant à dévier le faisceau horizontalement et donc à réaliser les lignes de l'image, d'un miroir M2 galvanométrique à 15 Hz servant à dévier le faisceau verticalement et donc à réaliser la trame de l'image ; et de deux systèmes afocaux de grandissement unitaire, AF1 situé entre les deux miroirs et AF2 situé après le miroir M2, ces systèmes afocaux étant utilisés pour conjuguer les plans de rotation des deux miroirs M1 et M2 avec le plan d'injection dans l'une des fibres. Selon l'invention, la vitesse de balayage est fixée pour permettre une observation des tissus in vivo in situ en temps réel. Pour cela, le balayage doit être suffisamment rapide pour qu'il y ait au moins 12 images / s affichées à l'écran pour un mode d'affichage de 640 x 640 pixels correspondant au mode le plus lent. Pour les modes d'affichage ayant moins de pixels, le nombre d'images acquises par seconde sera ainsi toujours supérieur à 12 images / s. En variante, les moyens de balayage peuvent comprendre notamment un miroir rotatif, des composants intégrés de type MEMs (miroirs de balayage X et Y), ou un système acousto-optique.

Le faisceau d'excitation dévié en sortie des moyens de balayage est dirigé vers les moyens optiques 5 afin d'être injecté dans l'une des fibres du guide d'image 6. Ces moyens 5 sont constitués ici de deux ensembles optiques E1 et E2. Le premier ensemble optique E1 permet de corriger en partie les aberrations optiques en bord de champ des moyens de balayage 4, l'injection étant ainsi optimisée sur l'ensemble du champ optique (au centre comme au bord). Le second ensemble optique E2 est destiné à réaliser l'injection proprement dite. Sa focale et son ouverture numérique ont été choisies pour optimiser le taux d'injection dans les fibres optiques du guide 6. Selon un mode de réalisation permettant d'obtenir le critère d'achromaticité, le premier ensemble E1 est constitué d'un doublet de lentilles, et le second ensemble E2 de deux doublets de lentilles suivi d'une lentille située près du guide d'image. En variante, cette optique d'injection pourrait être constituée de tout autre type d'optiques standards, comme par exemple deux triplets, ou de lentilles à gradient d'indice ou bien d'un objectif de microscope (toutefois plus coûteux).

Le guide d'image 6 est constitué d'un très grand nombre de fibres optiques souples (quelques dizaines de milliers), par exemple

30 000 fibres de 2 µm de diamètre et espacées de 3,3 µm. En pratique, on peut utiliser soit l'ensemble des fibres du guide d'image, soit un sous-ensemble choisi de ces fibres, par exemple centré.

En sortie de la fibre optique, le faisceau laser d'excitation est focalisé par la tête optique 7 dans l'échantillon 13 en un point 14 situé à une profondeur donnée située entre quelques dizaines de µm et une centaine de µm, par rapport à la surface 15 de l'échantillon au contact de laquelle est destinée à être placée la tête optique. Cette profondeur peut être par exemple de 40 µm. La tête optique permet donc de focaliser le flux sortant du guide d'image dans l'échantillon, mais également de collecter le flux de fluorescence revenant de l'échantillon. La tête optique possède un grandissement de 2,4 et une ouverture numérique sur l'échantillon de 0,5. Ces deux paramètres sont choisis afin que le signal de retour se fasse uniquement dans la fibre optique ayant transmis le signal d'excitation et non pas dans des fibres adjacentes et conserver ainsi le filtrage confocal à l'aide d'une fibre. Avec ces valeurs de grandissement et d'ouverture numérique, la résolution axiale est de l'ordre de 10 µm et la résolution latérale de l'ordre de 1,4 µm. L'ouverture numérique est également choisie de manière à optimiser le nombre de photons récupérés qui doit être le plus important possible. La tête optique peut être constituée d'optiques classiques (doublet, triplet, asphérique) et/ou de lentilles à gradient d'indice (GRIN) présentant une qualité optique et un chromatisme adaptés à la confocalité, c'est à dire minimisant les aberrations optiques, qui entraîneraient sinon notamment des dégradations sur la profondeur de champ et par conséquent sur la résolution axiale de l'appareillage. En fonctionnement, la tête optique est destinée à être posée au contact de l'échantillon 13. Ce dernier est un tissu biologique ou une culture cellulaire. L'expression de la fluorescence est réalisée par le bleu de méthylène qui re-émet des photons sur une bande spectrale de plusieurs dizaines de nanomètres à plus d'une centaine de manomètres.

Sur la voie de détection, le signal de fluorescence, en sortie du filtre de réjection 8, est ensuite focalisé par les moyens 9, constitués par exemple d'une lentille de détection, dans un trou de filtrage des moyens 10 de filtrage spatial. La focale de la lentille de détection est calculée pour que le signal de fluorescence provenant d'une fibre soit de la taille ou légèrement inférieure à celle du trou de filtrage. Ce dernier permet de conserver la lumière de fluorescence ne provenant que de la fibre illuminée par le faisceau incident. Il permet de rejeter la lumière qui aurait pu être couplée dans les fibres adjacentes à celle qui est illuminée. La taille du trou est calculée pour que l'image d'une fibre s'y inscrive parfaitement. Ici, elle est de 20 µm. Par ailleurs, dans un souci d'optimiser la quantité de photons traversant le trou de filtrage, et donc le flux détecté, les moyens de balayage 4, les moyens d'injection 5, les moyens de focalisation 7 de la tête optique, et les moyens de détection 8, 9 et 10 sont adaptés au fluorophore détecté : ces moyens sont choisis pour être suffisamment achromatiques pour collecter des photons sur la bande la plus large d'émission du fluorophore qui est le bleu de méthylène.

Les moyens de détection 11 ont une sensibilité maximale aux longueurs d'onde de fluorescence du bleu de méthylène. On peut utiliser par exemple une photodiode à avalanches (APD) ou bien un photomultiplicateur. D'une façon générale, on optimise toute la chaîne d'acquisition (tête optique, guide d'onde, détecteur, électronique et logiciel de traitement d'image) de façon à détecter quelques centaines de pico watts de fluorophore dans in intervalle d'environ 80 nanosecondes. On détecte ainsi peu de photons de bleu de méthylène avec un système fonctionnant en temps réel.

En outre, pour obtenir une image en temps réel, la bande passante est choisie de préférence pour optimiser le temps d'intégration du signal de fluorescence. Elle est de 2 MHz, ce qui correspond à la fréquence d'échantillonnage minimale du guide d'image avec un temps d'intégration optimisé sur chaque pixel.

Les moyens électroniques et informatiques 12 de commande, d'analyse et de traitement numérique du signal détecté et de visualisation comprennent les cartes suivantes:
- une carte de synchronisation 20 qui a pour fonctions :
- de commander de manière synchronisée le balayage, c'est-à-dire le mouvement des miroirs ligne M1 et trame M2 ;
- de connaître à tout instant la position du spot laser ainsi balayé ; et
- de gérer toutes les autres cartes par l'intermédiaire d'un microcontrôleur lui-même pouvant être piloté ;
- une carte détecteur 21 qui comprend un circuit analogique qui réalise notamment une adaptation d'impédance, un convertisseur analogique numérique puis un composant logique programmable (par exemple un circuit FPGA) qui met en forme le signal ;
- une carte d'acquisition numérique 22 qui permet de traiter un flot de données numériques à fréquence variable et de l'afficher sur un écran 23 ;
- une carte graphique 24.

En variante, on peut utiliser une seule carte regroupant les fonctionnalités de ces différentes cartes.

Le traitement d'image se fait de la manière suivante.

A la mise en place d'un guide d'image dans l'appareillage, une première opération est effectuée pour reconnaître le motif des fibres dans le guide d'image, et donc connaître l'emplacement réel de chaque fibre destinée à être utilisée.

Les opérations suivantes sont également réalisées préalablement à l'utilisation de l'appareillage :
- la détermination du taux d'injection propre à chaque fibre, à l'aide d'un échantillon homogène, ce taux d'injection pouvant varier d'une fibre à une autre ; et
- la mesure de l'image de fond, effectuée sans échantillon.

Ces deux opérations peuvent être réalisées régulièrement en fonction de la fréquence d'utilisation de l'appareillage. Les résultats obtenus vont être utilisés pour corriger en fonctionnement le signal numérique en sortie de la carte détecteur.

En fonctionnement, selon l'invention 2 groupes de traitements sont effectués sur le signal numérique en sortie de la carte détecteur :

Le premier groupe consiste dans un premier temps à corriger le signal numérique notamment pour tenir compte du taux propre réel d'injection de la fibre dont est issu ledit signal et pour lui soustraire la partie du flux correspondant à l'image de fond. Cela permet de ne traiter qu'un signal correspondant réellement à l'échantillon observé. On utilise pour ce groupe de traitement un algorithme de calcul classique qui est optimisable pour respecter le cas échéant la contrainte du temps réel.

Le second groupe consiste ensuite, à partir du signal corrigé, à reconstruire l'image numérique qui sera visualisée par le praticien. Le but du traitement effectué est d'offrir à la visualisation une image numérique reconstituée qui ne soit pas simplement une mosaïque d'éléments d'image correspondant chacun à un signal numérique corrigé d'une fibre mis côte à côte, mais d'offrir une image numérique reconstituée qui ne fasse plus apparaître les fibres. On utilise pour cela un algorithme destiné à effectuer un certain nombre d'opérations sur chaque pixel, l'algorithme étant choisi pour respecter la contrainte de temps réel, c'est à dire qu'il doit représenter un bon compromis entre la complexité des opérations demandées, la qualité du résultat que l'on peut obtenir et le temps de calcul. A titre d'exemple, on peut utiliser un algorithme de filtrage passe-bas Gaussien.

Le fonctionnement de l'appareillage est le suivant. La source 1 produit un faisceau parallèle circulaire d'excitation à λ=635 nm, qui est ensuite remis en forme dans le système afocal 2 afin de lui donner la taille adéquate pour la meilleure injection possible dans le coeur d'une fibre. Ce faisceau est ensuite envoyé vers le système de séparation dichroïque 3 qui réfléchit la longueur d'onde d'excitation. Le faisceau incident est ensuite dévié angulairement dans le temps dans les deux directions de l'espace par le système de balayage optomécanique de miroirs 4, et injecté grâce aux moyens optiques d'injection 5 dans l'une des fibres du guide d'image 6. Les moyens électroniques 12 servent à commander l'injection à un instant donné de l'une des fibres optiques du guide d'image en déviant angulairement le faisceau au moyen des miroirs, et ce point par point pour une ligne donnée, et ligne après ligne, pour constituer l'image. En sortie du guide, la lumière émergeant de la fibre injectée est focalisée dans l'échantillon grâce à la tête optique 7 en un point situé à une profondeur donnée située sensiblement entre quelques dizaines de µm et une centaine de µm. Grâce au balayage, l'échantillon est illuminé point par point. A chaque instant, le spot illuminant le tissu émet alors un signal de fluorescence qui a la particularité d'être décalé vers des plus grandes longueurs d'onde. Ce signal de fluorescence est capté par la tête optique 7, puis suit le chemin inverse du faisceau d'excitation jusqu'au filtre dichroïque 3 qui va transmettre le signal de fluorescence vers la voie de détection. Les réflexions parasites se produisant à la longueur d'onde d'excitation vont ensuite être rejetées par le filtre de réjection 8. Enfin, le signal de fluorescence est focalisé dans le trou de filtrage 10 pour ne sélectionner que la lumière provenant de la fibre excitée et les photons sont détectés par la photodiode à avalanche 11. Le signal détecté est ensuite numérisé et corrigé. Les signaux détectés, les uns après les autres, sont traités en temps réel grâce au traitement d'image décrit plus haut pour permettre la reconstruction d'une image en temps réel visualisée à l'écran.

La mise en oeuvre du procédé selon la présente invention, pour notamment l'observation du tissu humain peut se faire en limitant la concentration du Bleu de Méthylène dans le tissu : une faible concentration de l'ordre de 0.5%; en limitant la puissance du faisceau laser : une faible puissance de l'ordre de 10mW/µs; et en augmentant la sensibilité de détection du système de façon à détecter un maximum de fluorophore.

Bien sûr, l'invention n'est pas limitée aux exemples qui viennent d'être décrits et de nombreux aménagements peuvent être apportés à ces exemples sans sortir du cadre de l'invention. On peut notamment utiliser un système comprenant une seule fibre optique ou un "bundle" de fibres optiques, un balayage point à point ou ligne à ligne, un balayage distal ou proximal, avec ou sans tête optique, pour de la fluorescence ou de la réflectance.

## Revendications

1. Procédé d'acquisition d'imagerie de fluorescence, in vivo, d'un tissu au moyen d'un système d'acquisition comprenant au moins une fibre optique d'excitation du tissu par balayage d'un faisceau laser d'excitation, **caractérisé en ce qu'**on utilise ledit système pour la détection de signaux de fluorescence émis par du Bleu de Méthylène présent dans le tissu, et **en ce qu'**on adapte la puissance du faisceau laser d'excitation pour ne pas atteindre le seuil de phototoxicité du bleu de méthylène et **en ce que** cette puissance est inférieure ou égale à 10 mW pour une microseconde d'éclairage.

2. Procédé selon la revendication 1, **caractérisé en ce que** ledit système est un système d'endo-microscopie confocale.

3. Procédé selon l'une quelconque des revendications précédentes, **caractérisé en ce que** la longueur d'onde du faisceau lumineux d'excitation est comprise entre 600nm et 680nm.

4. Procédé selon l'une quelconque des revendications précédentes, **caractérisé en ce que** la concentration du Bleu de Méthylène est sensiblement égale à 0.5%.

5. Procédé selon l'une quelconque des revendications 1 à 3, **caractérisé en ce qu'**on utilise une concentration maximum du Bleu de Méthylène égale à 0.5%, et **en ce que** la puissance du faisceau laser d'excitation et la sensibilité dudit système sont adaptées de façon à détecter un signal de fluorescence de niveau suffisamment élevé pour permettre au système d'élaborer une image.

6. Procédé selon l'une quelconque des revendications précédentes, **caractérisé en ce que** ledit système est équipé d'un guide d'image constitué d'une pluralité de fibres optiques éclairées tour à tour, chaque fibre étant apte à véhiculer le signal d'excitation et le signal de fluorescence rétro-émis.

7. Procédé selon l'une quelconque des revendications précédentes, **caractérisé en ce que** le faisceau lumineux balaye le tissu de façon à acquérir en temps réel au moins douze images par seconde.

8. Application du procédé selon l'une quelconque des revendications précédentes pour l'acquisition d'images de noyaux cellulaires en endomicroscopie confocale.

## Patentansprüche

1. Verfahren zum Erfassen von Fluoreszenzbildern eines Gewebes in vivo mittels eines Akquisitionssystems, das zumindest eine optische Faser zum Anregen des Gewebes durch Abtasten mit einem Anregungslaserstrahl aufweist, **dadurch gekennzeichnet, dass** das System zum Erfassen von Fluoreszenzsignalen verwendet wird, die von im Gewebe vorhandenem Methylenblau ausgegeben werden, und dass die Leistung des Anregungslaserstrahls so abgestimmt wird, dass die Phototoxizitätsschwelle von Methylenblau nicht erreicht wird und dass diese Leistung kleiner oder gleich 10 mW bei einer Beleuchtung von einer Mikrosekunde ist.

2. Verfahren nach Anspruch 1, **dadurch gekennzeichnet, dass** das System ein konfokales, endomikroskopisches System ist.

3. Verfahren nach einem der vorangehenden Ansprüche, **dadurch gekennzeichnet, dass** die Wellenlänge des Anregungslichtstrahls zwischen 600 nm und 680 nm beträgt.

4. Verfahren nach einem der vorangehenden Ansprüche, **dadurch gekennzeichnet, dass** die Konzentration von Methylenblau im Wesentlichen gleich 0,5 % ist.

5. Verfahren nach einem der Ansprüche 1 bis 3, **dadurch gekennzeichnet, dass** eine maximale Konzentration von Methylenblau gleich 0,5 % verwendet wird und dass die Leistung des Anregungslaserstrahls und die Empfindlichkeit des Systems so abgestimmt werden, dass ein Fluoreszenzsignal erfasst wird, dessen Pegel hoch genug ist, um dem System zu gestatten, ein Bild zu erstellen.

6. Verfahren nach einem der vorangehenden Ansprüche, **dadurch gekennzeichnet, dass** das System mit einem Bildleiter ausgestattet ist, der aus einer Mehrzahl von optischen Fasern besteht, die der Reihe nach beleuchtet werden, wobei jede Faser dazu geeignet ist, das Anregungssignal und das rückgesendete Fluoreszenzsignal zu übertragen.

7. Verfahren nach einem der vorangehenden Ansprüche, **dadurch gekennzeichnet, dass** der Lichtstrahl das Gewebe so abtastet, dass er in Echtzeit zumindest zwölf Bilder pro Sekunde erfasst.

8. Anwendung des Verfahrens nach einem der vorangehenden Ansprüche zum Erfassen von Bildern von Zellkernen durch konfokale Endomikroskopie.

## Claims

1. Method for the *in-vivo* acquisition of fluorescence imaging of a tissue using an acquisition system comprising at least one optical fibre for exciting the tissue by scanning of an excitation laser beam, **characterized in that** said system is used for the detection of fluorescence signals emitted by Methylene Blue present in the tissue, and **in that** the power of the excitation laser beam is adapted so as not to reach the phototoxicity threshold of Methylene Blue and **in that** this power is less than or equal to 10 mW for one microsecond of illumination.

2. Method according to claim 1, **characterized in that** said system is a confocal endomicroscopy system.

3. Method according to any one of the preceding claims, **characterized in that** the wavelength of the excitation light beam is comprised between 600 nm and 680 nm.

4. Method according to any one of the preceding claims, **characterized in that** the concentration of Methylene Blue is approximately equal to 0.5%.

5. Method according to any one of claims 1 à 3, **characterized in that** a maximum concentration of Methylene Blue equal to 0.5% is used, and **in that** the power of the excitation laser beam and the sensitivity of said system are adapted so as to detect a fluorescence signal at a sufficiently high level to allow the system to produce an image.

6. Method according to any one of the preceding claims, **characterized in that** said system is equipped with an image guide constituted by a plurality of optical fibres illuminated in turn, each fibre being capable of conveying the excitation signal and the back-emitted fluorescence signal.

7. Method according to any one of the preceding claims, **characterized in that** the light beam scans the tissue so as to acquire at least twelve images per second in real time.

8. Application of the method according to any one of the preceding claims for the acquisition of images of cell nuclei in confocal endomicroscopy.
